Europäisches Patentamt

European Patent Office

Office européen des brevets

(19)

(11) Publication number: 0 177 342
A2

# (12) EUROPEAN PATENT APPLICATION

(21) Application number: 85307043.1

(22) Date of filing: 02.10.85

(51) Int. Cl.⁴: **A 61 K 37/36**
A 61 K 47/00, A 61 K 9/22

(30) Priority: 04.10.84 US 658082

(43) Date of publication of application:
09.04.86 Bulletin 86/15

(84) Designated Contracting States:
AT BE CH DE FR GB IT LI LU NL SE

(71) Applicant: GENENTECH, INC.
460 Point San Bruno Boulevard
South San Francisco California 94080(US)

(72) Inventor: Moore, Jerome Allen
404 Midway Avenue
San Mateo California 94402(US)

(72) Inventor: Ross, Michael Jay
1065 Hayne Road
Hillsborough California 94010(US)

(74) Representative: Armitage, Ian Michael et al,
MEWBURN ELLIS & CO. 2/3 Cursitor Street
London EC4A 1BQ(GB)

(54) Oral formulation of therapeutic proteins.

(57) Disclosed are novel pharmaceutically acceptable formulations uniquely designed for purpose of administering certain therapeutic amphophilic proteins in oral dosage form. One preferable formulation contains biologically active human growth hormone, mineral oil and sodium salicylate. Also disclosed is the technology associated with preparing such preparations and data supporting gastrointestinal tract absorption of the therapeutic protein.

EP 0 177 342 A2

Croydon Printing Company Ltd.

-1-

## ORAL FORMULATION OF THERAPEUTIC PROTEINS

The present invention relates to the pharmaceutical preparation of polypeptides of therapeutic value which enables them to be absorbed through the wall of the gastrointestinal (GI) tract after oral administration. The invention applies to human as well as veterinary applications.

From the viewpoint of the veterinary practitioner or in the case of a human patient's viewpoint, oral administration of therapeutic agents is often preferable to parenteral administration. However, oral delivery of many such agents is considered to require unacceptably large dosages for effective absorption. This is a particular problem for agents which are not available in large quantities. One attempt to improve the oral absorption of polar, bioactive, therapeutic substances is described in European Patent Application Publication No. 36145, published September 23, 1981 (the EP application). It discloses the addition to a wide variety of bioactive agents, including polypeptides, of many different adjuvants, including hydroxyaryl or hydroxyaralkyl acids, such as salicylic acid and its salts. When required, the EP application suggests the addition of suitable binders, lubricants, disintegrating agents, and coloring agents. Noticeably absent from the list of therapeutic substances are polypeptides larger than insulin (molecular weight of about 6000). Such larger polypeptides would be a potentially important class of therapeutic agents for oral administration. The advent of biotechnology has made a number of hitherto unavailable proteins of therapeutic value capable of being produced.

The reason for this lack of disclosure of oral administration of large peptides can be inferred from an article by three authors including the two named inventors of the EP application, but published long after filing the application (Caldwell, L.T., et al. _Pharmaceutical_ _Technology_, 50 (Oct. 1983)). There, it is stated that oral administration of most polypeptides are subject to the problems of (1) chemical and enzymatic breakdown and (2) an inadequate rate of absorption to provide useful plasma concentrations. To meet these problems the article espouses the use of a rectal dosage form even though it is less desirable from the patient's viewpoint. At page 54, the article specifies insulin as a target compound for rectal administration because of tradition and because the authors consider the molecule to be "quite large" (implicitly too large for oral administration). This latter article by the named EP application inventors does not suggest that even rectal administration would be effective for substantially larger polypeptides, perhaps because the authors believed that the inadequate rate of absorption referred to at page 50 of their article would preclude the use of large polypeptides even for rectal absorption.

In accordance with the present invention there is provided a formulation in which a therapeutic amphophilic protein of MW > 12,000 daltons is administered in oral dosage form to a human or animal subject. The oral dosage preferably includes one component serving as a lipophilic vehicle, preferably mineral oil, and a GI tract absorption enhancing agent, (e.g., sodium salicylate). It has been found that the combination of these two components produces synergistic effects in the absorption of amphophilic proteins in the ileum and colon.

## Brief Description of the Drawings

Fig. 1 illustrates a plot of hGH concentration in serum versus time for intravenously injected animals.

Fig. 2 illustrates absorption of hGH versus time following injection into the ileum.

Fig. 3 illustrates absorption of hGH versus time following injection into the colon.

## Description of Preferred Embodiments

The present invention is applicable to administration of amphophilic proteins of therapeutic value in oral dosage form. The proteins should be greater than twice the size of insulin (i.e., about 12,000 daltons) and have a composition of amino acids which leads to regions of hydrophilic as well as hydrophobic character in the molecule. The proteins should be of therapeutic value when administered parenterally. For human use they include any of the following proteins or "structurally similar bioactive equivalents": human growth hormone, human alpha interferon, human gamma interferon, human tissue plasminogen activator, human tumor necrosis factor and the like. If designed for use in animals, the proteins might include bovine growth hormone, bovine alpha interferon, bovine gamma interferon, porcine growth hormone, chicken growth hormone, and the like.

The present invention is particularly applicable to the administration to a human subject in growth phase of human growth hormone (hGH) or its structurally similar bioactive equivalent, in oral dosage form. The hGH may be derived from natural sources such as human pituitaries as described in Roos, P. et al., Biochem. Biophys. Acta. 75:525 (1963). Another source of the hGH is via vectors constructed using recombinant DNA techniques as embodied in U.S. Patent 4,342,832 - See Goeddel et al., Nature 281, 544 (1979).

By "structurally similar bioactive equivalent" is meant a peptide with an amino acid sequence which, although not identical to that of naturally occurring hGH, is sufficiently similar in structure to produce substantially equivalent effects on the subject's growth phase to that produced by hGH itself.

Unexpectedly, it has been found that an oral dosage form of hGH, a relatively large protein (molecular weight of about 22,000 daltons) can be formulated with adjuvants which substantially increase absorption in the GI tract.

This provides a technique for overcoming the problem of mucosal impermeability which likely had discouraged the use of polypeptides in oral dosage form.

It has been found that absorption is synergistically enhanced in the GI tract of methionyl hGH produced in accordance with the aforementioned Goeddel paper when the hGH is formulated with a combination of adjuvants, specifically sodium salicylate and mineral oil.

Broadly stated, the oral dosage form of hGH includes component (a) - a lipophilic vehicle, and component (b) - a GI tract absorption enhancing agent. Component (a) is characterized by lipophilic character, immiscibility with water, and compatability with hGH (or other active agents set out infra) and with capability of use in enterically administered pharmaceuticals. Suitable lipophilic vehicles include mineral oil, triglycerides, esterified glycols, polyglycols with hydrophobic alkyl side chains, phospholipids, and sterols. Mineral oil, USP, is particularly effective. Due to their similar lipophilic properties, it is believed that the remaining compounds in the above group would also be suitable for assisting the enhancement of GI tract absorption when used in combination with component (b).

A component (b) includes hydroxyaryl or hydroxyaralkyl acids or their salts, esters, or amides. Sodium salicylate has been found to be effective for this purpose. Other compounds with similar properties include other salicylic acid derivatives and amines of 1, 3 dicarbonyl compounds and enamino acids, and their salts, amides and esters.

Although the theoretical mechanism for enhancement of GI tract absorption is not known with precision, it is believed that the following theory is applicable. In order for large proteins to be absorbed into either the blood stream or the lymphatic system and thus to be bioavailable, they need to either pass between cells lining the gut wall, or be transported through lipid membranes either by active or passive transport. It is thought that the combination of components (a) and (b) are required. Component (a) is thought to change the characteristics of the mucosal barrier between the intestinal wall and the intestinal contents as well as affecting the transport mechanisms through the cells in the intestinal wall. It serves as a lipophilic carrier. In combination, components (a) and (b) serve to provide effective transport of large intact proteins into the blood stream or lymphatic system. It is the amphophilic or combined hydrophilic/hydrophobic nature of these proteins which causes both the absorption enhancer and the lipophilic molecule to be needed.

The ratio of component (a) and component (b) in the formulation is not critical. It is preferable that there be at least about one unit of component (a) per unit protein to be administered. Of course, the dosage of protein to be administered is the therapeutically effective amount to be determined in accordance with well established medical practice based upon the individual patient.

One potential obstacle to oral administration of the protein is the low gastric pH level and the presence of proteolytic enzymes in the upper GI tract which could inactivate the protein before it can be absorbed by the intestinal mucosa. This problem can be solved by the use of component (a) in combination with enteric coatings which are available for tablets and capsules that can be made to release the drug at sites downstream in the intestine (e.g., the ileum and colon) thus avoiding such harsh gastric conditions and even many of the proteolytic enzymes and bile salts present in the upper small intestine. Suitable enteric coatings of this type include cellulose acetate phthalates as set forth in Remington's Pharmaceutical Sciences (13th & 16th editions).

Component (b) may be dissolved or suspended in component (a) (mineral oil) which is in a liquid form and contained by an appropriate outer shell. A suitable shell comprises a capsule formed of gelatin or the like as set forth in Modern Pharmaceutics, 412 (1979). Outer shells for formulation may also be employed in accordance with known practice. Alternatively, they are contained in a controlled release oral dosage form such as described in Theeuwes, F., Drug Dev. and Ind. Pharm. 9 (7), 1331 (1983). Although the present invention is particularly effective for hGH, it is also believed to be effective for oral administration of certain other active agents in oral dosage form such other active agents are amphophilic proteins having therapeutic value, a defined globular structure, and a molecular weight greater than 12,000 daltons. Suitable active agents for human subjects includes, in addition to hGH, human alpha interferon, human gamma interferon, human tissue plasminogen activator, human tumor necrosis factor, and structurally similar bioactive equivalents of any of said active agents. Suitable active agents for animals include bovine growth hormone, bovine alpha interferon, bovine gamma interferon, porcine growth hormone, chicken growth hormone, and structurally similar bioactive equivalents of any of said active agents.

The following example illustrates the dramatic effect of using sodium salicylate and mineral oil adjuvants in combination with methionyl hGH produced using recombinant DNA methods. Specifically, the hGH was produced by methods described in Goeddel et al and Roos et al.

Example

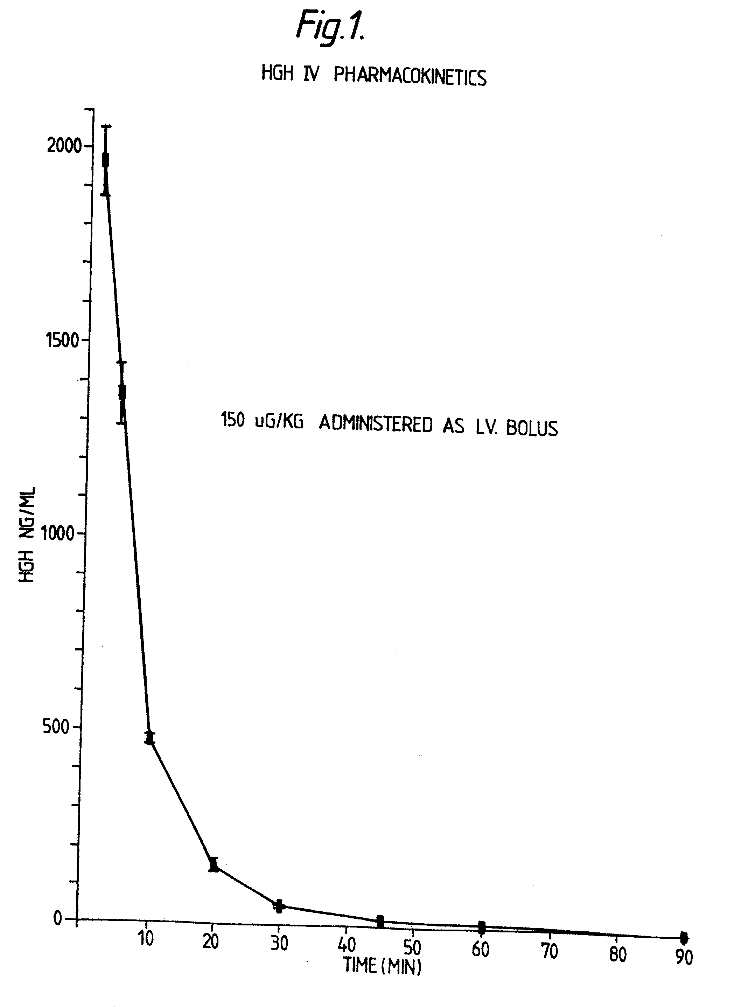

The aforementioned hGH was placed into an injection dosage form including 0.1 ml hGH (0.15 mg/ml) in aqueous mannitol phosphate buffer. To provide a basis of comparison, the above formulation was first injected into male CD rats weighing approximately 350 grams. The rats were fasted over night and anesthetized with rompun (4 mg/kg) intramuscularly followed 20 minutes later by ketamine (40 mg/kg) and acepromazine (1 mg/kg) intramuscularly. Fig. 1 illustrates the serum concentration versus time plot for the intravenously injected animals. Six animals were used for the intravenous injections. Blood samples were collected at 2.5, 5, 10, 20, 30, 45, 60 and 90 minutes after injection.

The area under the curve computed by the trapezoidal rule is 14,631 ng-min/ml. The results shown in Figure 1 are used to compute bioavailability for the intestinal administration treatment groups. The dose used in intravenous injection was only 5% of that used in intestinal administration set out below and so this is taken into account in computing bioavailability.

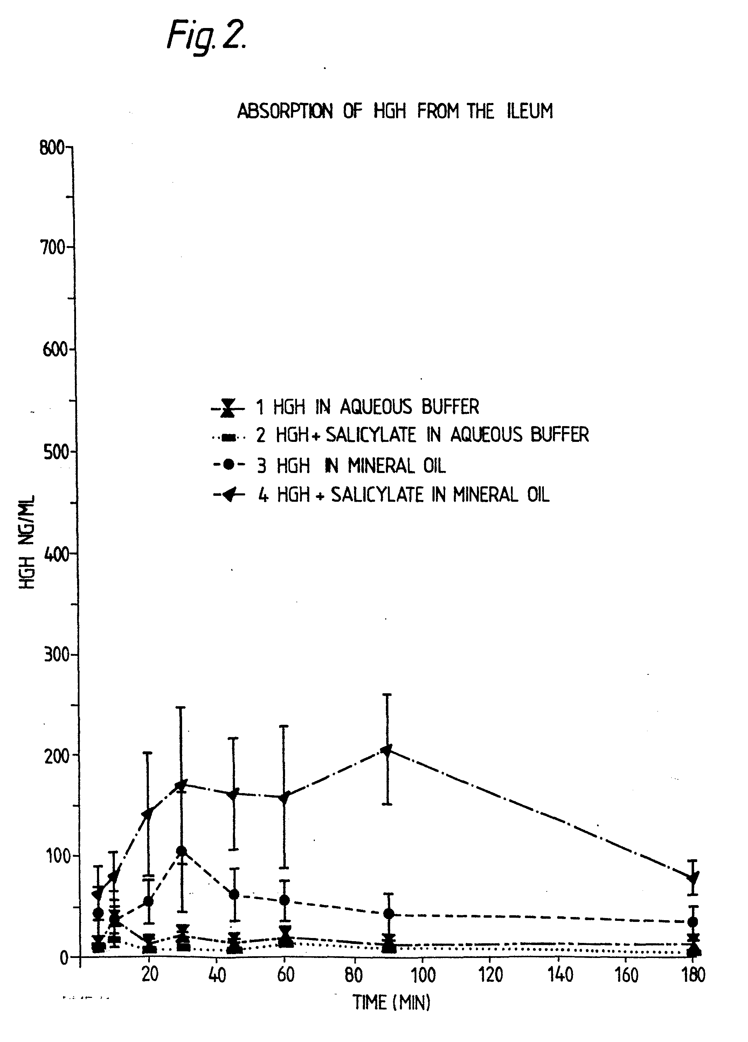

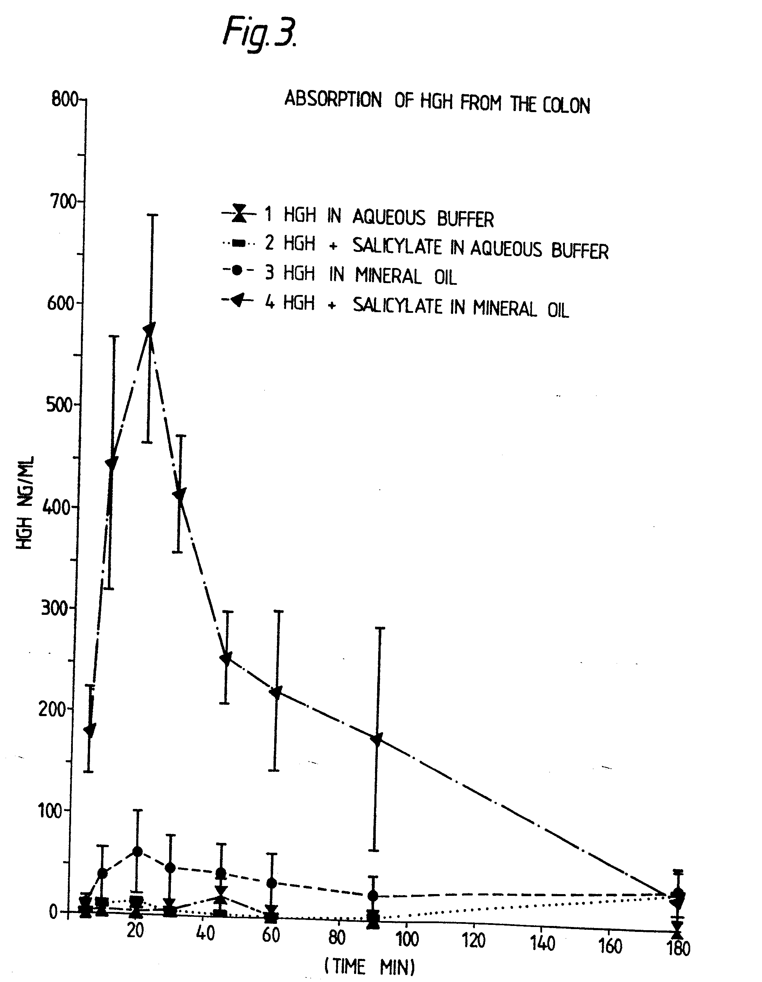

Four different formulations of adjuvants were used for injecting hGH into four different areas of the GI tract, the stomach, the duodenum, the ileum and the colon. Blood samples were taken at 5, 10, 20, 30, 45, 60 and 90 minutes after injection. Serum concentrations of hGH were determined by radioimmunoassay. While there was some improvement in enhancement of absorption of hGH in the stomach and duodenum using combination with both mineral oil and sodium salicylate, such enhancement was particularly dramatic in the ileum and colon as illustrated in Figs. 2 and 3 respectively. Six animals were used for each formulation at each injection site. The following formulations were injected in each site (1) growth hormone (5 mg/ml) in an aqueous buffer (0.2 ml.); (2) growth hormone (5 mg/ml) plus sodium salicylate (40 mg/ml) in an aqueous buffer (0.2 ml); (3) growth hormone (5 mg/ml) in (0.2 ml) mineral oil; and (4) growth hormone (5 mg/ml) plus sodium salicylate (40 mg/ml) in 0.2 ml mineral oil. As illustrated in Figs. 2 and 3, there is a substantial absorption enhancement in the ileum and colon using sodium salicylate in mineral oil base. It can be concluded that the absorption of hGH from these sections of the intestine must have been sustained for at least ninety minutes since serum levels are still relatively high for that period. If absorption were significantly more rapid, the level would drop off faster based upon the serum half life indicated by the intravenous data showed in Fig. 1. Bioavailability based on area under serum concentration versus time curve relative to intravenous administration is illustrated in the following table in which the numbers correspond to the above formulation:

TABLE 1

|   | STOMACH | DUODENUM | ILEUM | COLON |
|---|---------|----------|-------|-------|
| 1 | 1.0 | 1.3 | 0.9 | 0.2 |
| 2 | 0.6 | 0.5 | 0.6 | 0.8 |
| 3 | 1.2 | 3.7 | 2.9 | 2.1 |
| 4 | 1.2 | 2.2 | 8.9 | 12.8 |

The data in Table I describe in numerical terms what was illustrated in Figs. 2 and 3 with respect to the ileum and colon, namely that there was a synergistic interaction with respect to absorption in the ileum and colon of hGH in the presence of salicylate and mineral oil in comparison to that of either adjuvant alone.

The results of the above test show that there is sufficient enhancement of absorption of hGH from the ileum and colon that the product could be a useful oral dosage form.

CLAIMS

1. An oral dosage formulation including (i) an active agent comprising an amphophilic protein having therapeutic value and a molecular weight of greater than 12,000 daltons, (ii) a vehicle which is lipophilic, immiscible in water, and compatible with said active agent and usable in enterically administered pharmaceuticals, and (iii) a GI tract absorption enhancing agent selected from hydroxyaryl or hydroxyarylalkyl acids, the salts, esters or amides of said acids, and enamine derivatives of 1, 3 dicarbonyl compounds and amino acids and their salts, amides, and esters.

2. A formulation of claim 1 in which said active agent comprises human growth hormone or its structurally similar bioactive equivalent.

3. A formulation of claim 1 in which said active agent is selected from human growth hormone, human alpha interferon, human gamma interferon, human tissue plasminogen activator, human tumor necrosis factor, and structurally similar bioactive equivalents of any of said active agents.

4. A formulation of claim 1 in which said active agent is selected from bovine growth hormone, bovine alpha interferon, bovine gamma interferon, porcine growth hormone, chicken growth hormone, and structurally similar bioactive equivalents of any of said active agents.

5. A formulation of any one of the preceding claims in which said lipophilic vehicle is selected from mineral oil, triglycerides, esterified glycols, esterified polyglycols, polyglycols with hydrophobic alkyl side chains, phospholipids, and sterols.

6.     A formulation of any one of claims 1 to 4 in which said lipophilic vehicle comprises mineral oil.

7.     A formulation of any one of the preceding claims in which said oral dosage also includes a GI tract absorption enhancing agent having calcium chelating properties.

8.     A formulation of any one of claims 1 to 6 in which said absorption enhancing agent comprises sodium salicylate.

9.     A growth enhancing formulation comprising hGH or its structurally similar bioactive equivalent, in a therapeutically effective amount, in oral dosage form.

10.     A formulation of claim 9 in which said oral dosage includes an enteric coating.

11.     A formulation of claim 9 or claim 10 in which said oral dosage is in a controlled release form.

12.     A formulation of any one of claims 9, 10 and 11 in which said oral dosage comprises a vehicle which is lipophilic, immiscible in water, compatible with said active agent and usable in enterically administered pharmaceuticals.

13.     A formulation of claim 12 in which said lipophilic vehicle is selected from mineral oil, triglycerides, esterified glycols, esterified polyglycols, derivativized polyglycols, phospholipids, and sterols.

14.     A formulation of claim 12 in which said lipophilic vehicle comprises mineral oil.

15.     A formulation of any one of claims 9 to 14 in which said oral dosage also includes a GI tract absorption enhancing agent having calcium chelating properties.

16. A formulation of any one of claims 9 to 14 which said oral dosage includes a combination of additives comprising mineral oil and sodium salicylate.

1

## CLAIMS

1.    A process comprising the manufacture of an oral dosage formulation including (i) an active agent comprising an amphophilic protein having therapeutic value and a molecular weight of greater than 12,000 daltons, (ii) a vehicle which is lipophilic, immiscible in water, and compatible with said active agent and usable in enterically administered pharmaceuticals, and (iii) a GI tract absorption enhancing agent selected from hydroxyaryl or hydroxyarylalkyl acids, the salts, esters or amides of said acids, and enamine derivatives of 1, 3 dicarbonyl compounds and amino acids and their salts, amides, and esters.

2.    A process of claim 1 in which said active agent comprises human growth hormone or its structurally similar bioactive equivalent.

3.    A process of claim 1 in which said active agent is selected from human growth hormone, human alpha interferon, human gamma interferon, human tissue plasminogen activator, human tumor necrosis factor, and structurally similar bioactive equivalents of any of said active agents.

4.    A process of claim 1 in which said active agent is selected from bovine growth hormone, bovine alpha interferon, bovine gamma interferon, porcine growth hormone, chicken growth hormone, and structurally similar bioactive equivalents of any of said active agents.

5.    A process of any one of the preceding claims in which said lipophilic vehicle is selected from mineral oil, triglycerides, esterified glycols, esterified polyglycols, polyglycols with hydrophobic alkyl side chains, phospholipids, and sterols.

6. A process of any one of claims 1 to 4 in which said lipophilic vehicle comprises mineral oil.

7. A process of any one of the preceding claims in which said oral dosage also includes a GI tract absorption enhancing agent having calcium chelating properties.

8. A process of any one of claims 1 to 6 in which said absorption enhancing agent comprises sodium salicylate.

9. A process comprising the manufacture of a growth enhancing formulation comprising hGH or its structurally similar bioactive equivalent, in a therapeutically effective amount, in oral dosage form.

10. A process of claim 9 in which said oral dosage includes an enteric coating.

11. A process of claim 9 or claim 10 in which said oral dosage is in a controlled release form.

12. A process of any one of claims 9, 10 and 11 in which said oral dosage comprises a vehicle which is lipophilic, immiscible in water, compatible with said active agent and usable in enterically administered pharmaceuticals.

13. A process of claim 12 in which said lipophilic vehicle is selected from mineral oil, triglycerides, esterified glycols, esterified polyglycols, derivativized polyglycols, phospholipids, and sterols.

14. A process of claim 12 in which said lipophilic vehicle comprises mineral oil.

15.  A process of any one of claims 9 to 14 in which said oral dosage also includes a GI tract absorption enhancing agent having calcium chelating properties.

16.  A process of any one of claims 9 to 14 which said oral dosage includes a combination of additives comprising mineral oil and sodium salicylate.

Fig.1.

HGH IV PHARMACOKINETICS

150 uG/KG ADMINISTERED AS I.V. BOLUS

# Fig. 2.

ABSORPTION OF HGH FROM THE ILEUM

## Fig.3.

ABSORPTION OF HGH FROM THE COLON

—✕— 1 HGH IN AQUEOUS BUFFER
···■··· 2 HGH + SALICYLATE IN AQUEOUS BUFFER
—●— 3 HGH IN MINERAL OIL
—◀— 4 HGH + SALICYLATE IN MINERAL OIL

HGH NG/ML

(TIME MIN)